# EUROPÄISCHE PATENTSCHRIFT

(11) **EP 4 558 088 B1**
(45) Veröffentlichungstag und Bekanntmachung des Hinweises auf die Patenterteilung: **03.06.2026**
(21) Anmeldenummer: 24717223.2
(22) Anmeldetag: 09.04.2024
(51) Int. Cl.: A61C 7/36, A61C 19/06, A61H 1/02, A61F 5/56

(54) **ZAHNAUFSATZKISSEN**
DENTAL BITE CUSHION
COUSSINET DE FIXATION DENTAIRE

(30) Priorität: 26.04.2023 DE 202023102233 U
(43) Veröffentlichungstag der Anmeldung: 28.05.2025
(73) Patentinhaber: TJ - Motion GmbH, 91085 Weisendorf (DE)
(72) Erfinder: BREHM, Peter, 91085 Weisendorf (DE); LYER, Sonja, 64297 Darmstadt (DE)
(74) Vertreter: Grünecker Patent- und Rechtsanwälte PartG mbB
(86) Internationale Anmeldenummer: PCT/EP2024/059568
(87) Internationale Veröffentlichungsnummer: WO 2024/223285

(56) Entgegenhaltungen:
- US-A- 4 304 227
- US-A- 5 795 150
- US-B2- 7 234 467

## Beschreibung

Die vorliegende Erfindung betrifft ein Zahnaufsatzkissen, das insbesondere für eine Anwendung durch einen Benutzer zu einer Entlastungs- und Entspannungsbehandlung der Kiefermuskulatur und sanfte Dehnung des Kiefergelenkes geeignet ist.

Es ist bereits bekannt, Zahnaufsatzkissen oder ähnliches dazu zu verwenden, beim Zusammenbeißen und Schließen des Kiefers bei Personen einen elastischen Druckabsorber zwischen den Zähnen vorzusehen, um insbesondere beim Pressen, Beißen und Knirschen einen Schutz der Zähne zu bewirken.

Nach dem Stand der Technik wurden gemäß der US 5836761 A elastische Dämpfer zum Einsetzen zwischen den Zähnen zwischen Oberkiefer und Unterkiefer eines Benutzers, insbesondere für Sportler, vorgesehen, um bei erhöhter Anstrengung und einem einhergehenden Zusammenbeißen der Zähne eine Entspannung für den Benutzer zu erzielen. Es wurde festgestellt, dass durch den Einsatz geeigneter Dämpfungselemente zur Ermöglichung des Pressens und Beißens (umgangssprachlich auch "Zähnefletschen" genannt) beim Benutzer ein Stressabbau möglich ist. US7234467B2 zeigt ein Zahnaufsatzkissen gemäß dem Oberbegriff des Anspruchs 1.

Es ist die Aufgabe der vorliegenden Erfindung, ein besonders vorteilhaftes Zahnaufsatzkissen bereitzustellen, mit dem in optimaler Weise eine Entspannungssituation beim Benutzer herbeiführbar ist.

Erfindungsgemäß wird diese Aufgabe gelöst durch ein Zahnaufsatzkissen mit einer zum Aufsatz auf einen oder mehrere nebeneinanderliegende Zähne eines Benutzers geeignet ausgebildeten Form und Größe, mit einem zur Auflage auf einer Zahnoberfläche des einen oder der mehreren Zähne ausgebildeten Grundkörper und zwei seitlichen, vom Grundkörper ausgehenden, und sich über die Länge des Grundkörpers erstreckenden Seitenwangen, die sich jeweils zu beiden Seiten des Grundkörpers erstrecken, und die derart angeordnet sind, dass sich ein zur Aufnahme eines oder mehrerer nebeneinanderliegender Zähne geeigneter Graben zu beiden Seiten des Grundkörpers zwischen den Seitenwangen und dem Grundkörper ergibt, wobei der Grundkörper eine Länge entlang der Richtung des Grabens von mehr als 20 mm und bis zu 35 mm aufweist.

Auf Grund der erhöhten Länge des erfindungsgemäßen Zahnaufsatzkissens werden ein oder mehrere Zähne des Benutzers bei Gebrauch über eine vergrößerte Fläche und Länge überdeckt, was zu einer stabileren Positionierung des Zahnaufsatzkissens und einer gleichmäßigeren und sanfteren Druckverteilung auf die Zähne beim Schließen des Kiefers führt. Die beidseitig am Grundkörper durch die Seitenwangen gebildeten Grabenstrukturen erhöhen die Positionierfähigkeit und Positionsstabilität und erleichtern die Ausrichtung der Zahnaufsatzkissen auf den Zähnen des Benutzers.

Das erfindungsgemäße Zahnaufsatzkissen ist insbesondere aus besonders weichem Material, vorzugweise medizinischem Silikon mit einer Shore A Härte von unter 30, besonders bevorzugt zwischen 18 und 22 gefertigt. Damit vermitteln die Zahnaufsatzkissen eine besonders angenehme Empfindung des Benutzers und schaffen dennoch einen physiologisch bedeutenden Entspannungseffekt, da der Pressdruck durch die Zähne vermindert wird. Das erfindungsgemäße Zahnaufsatzkissen kann auch dann einen vorteilhaften Effekt für den Nutzer aufweisen, wenn es aus Material, vorzugsweise medizinisches Silikon, mit einer Shore A Härte in Bereich von 30 bis 70 aufweist, vorzugsweise mit einer Shore A Härte von 45 bis 55.

Weiter ist bevorzugt, das Zahnaufsatzkissen auf besondere Weise physiologischen Gestaltungen nachzubilden, indem in der Natur vorkommende Größenverhältnisse verwendet werden. Die Größen der Höhe der Seitenwangen zu deren Länge, bzw. zur Länge des Grundkörpers, verhalten sich dabei wie ca. 1.61. Dieser Wert entspricht dem sogenannten "Goldenen Schnitt", oder den Verhältnissen aufeinander folgender (höherer) Glieder der Fibonacci Folge, die in der Natur vorkommende Wachstumsprozesse beschreibt.

Vorteilhafte Weiterbildungen werden durch Zahnaufsatzkissen gemäß den Schutzansprüchen 2 bis 8 bereitgestellt.

Nachfolgend wird die Erfindung beispielhaft anhand der begleitenden beiliegenden Zeichnungen genauer erläutert und beschrieben. In den begleitenden Zeichnungen zeigt:
Fig. 1 ein erfindungsgemäßes Zahnaufsatzkissen in 3-dimensionaler Ansicht,
Fig. 2 eine Seitenansicht des erfindungsgemäßen Zahnaufsatzkissens und
Fig. 3 eine Vorderansicht des erfindungsgemäßen Zahnaufsatzkissens.

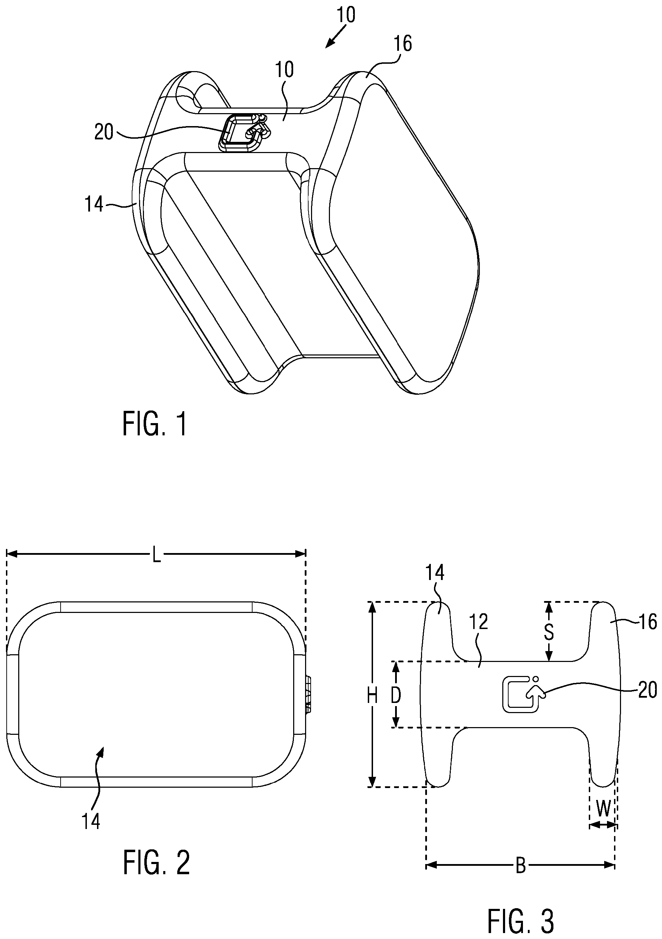

Wie mit Bezug auf die Fig. 1 bis Fig. 3 veranschaulicht ist, weist das erfindungsgemäße Zahnaufsatzkissen 10 einen geeigneten, blockförmigen Grundkörper 12 auf, der eine ausreichende Dicke D besitzt, um, wenn das Zahnaufsatzkissen auf einen oder mehrere Zähne des Benutzers aufgesetzt ist, beim Verschluss des Unterkiefers gegen den Oberkiefer eine sanfte und entspannende Dehnung in der Kiefermuskulatur im Bereich des Kiefergelenks zu erzielen. Die Dicke D des Grundkörpers, der zwischen den Zahnreihen des Ober- und des Unterkiefers liegt, beträgt dabei bei einem für Erwachsene und Kinder ab 8 Jahren geeigneten Zahnaufsatzkissen zwischen 2 und 8 mm, vorzugsweise zwischen 3 und 7 mm, und besonders bevorzugt zwischen 5 und 6 mm.

Das Zahnaufsatzkissen 10 gemäß der vorliegenden Erfindung weist eine Länge L des Grundkörpers 12 von mehr als 20 bis zu 35 mm auf. Besonders bevorzugt ist eine Länge L des Grundkörpers von 22 bis 28 mm, insbesondere von 24 bis 26 mm. Im Ausführungsbeispiel beträgt die Länge L 25 mm.

Das erfindungsgemäße Zahnaufsatzkissen 10 weist außerdem beidseitig Seitenwangen 14, 16 auf, die zusammen mit dem Grundkörper zu beiden Seiten des Grundkörpers jeweils einen Graben bilden, in die beim Einsatz beim Patienten die Aufnahme der Zähne des Oberkiefers und des Unterkiefers möglich ist. Damit lassen sich die erfindungsgemäßen Zahnaufsatzkissen 10 leicht auf einen oder mehrere Zähne des Benutzers auflegen und werden aufgrund ihrer Form beim Schließen des Mundes an den Zähnen sowohl des Oberkiefers als auch des Unterkiefers gehalten. Die Seitenwangen verhindern dabei ein Abkippen oder Abrutschen der Zahnaufsatzkissen zur Seite, wobei die Seitenwangen so ausgestaltet sind, dass eine Irritation des Zahnfleisches vermieden wird. Die Seitenwangen 14, 16 weisen dabei bevorzugt eine relativ geringe Wangenhöhe S von 3 bis 10 mm, bevorzugt 4 bis 7 mm, besonders bevorzugt von 5 bis 6 mm, im Ausführungsbeispiel von 5 mm auf. Damit zeichnen sich die Zahnaufsatzkissen bei einem Erwachsene und Kinder ab 8 Jahren dadurch aus, dass lediglich der Zahnhals des Benutzers durch die Seitenwangen abgedeckt wird ohne einen Druck auf das Zahnfleisch auszuüben und dadurch keine Irritationen auszulösen. Die Dicke W der Seitenwangen 14, 16 eines für Erwachsene und Kinder ab 8 Jahren geeigneten Zahnaufsatzkissens liegt im Bereich von 1 bis 5 mm, bevorzugt zwischen 1,5 und 3 mm, um ausreichend Elastizität zur einfachen Halterung des Zahnaufsatzkissens auf dem Backenzahn des Benutzers bereitzustellen. Bei der Bezeichnung der Dicke der sich zu Ihrem äußeren Ende hin verjüngenden Seitenwangen handelt es sich insbesondere um einen über die vom Grundkörper vorstehende Länge gemittelten Wert.

An der Stirnfläche des Grundkörpers kann ein Ornament 20 in Form einer Materialerhebung, beispielsweise in der Gestalt eines Markenkennzeichens, vorgesehen sein.

Als Material für das erfindungsgemäße Zahnaufsatzkissen 10 wird vorzugsweise für Lebensmittel- und Babyprodukte geeignetes Silikon verwendet. Das geeignete Material besitzt insbesondere eine Shore-A-Härte zwischen 10 und 30, vorzugsweise zwischen 15 und 25, und besonders bevorzugt zwischen 18 und 22, und im Ausführungsbeispiel von 20. Als Material für das Zahnaufsatzkissen eignet sich auch ein Material, beispielsweise medizinisches Silikon, mit einer Shore A Härte von 30 bis 70, vorzugsweise von 50 +- 5.

Das Produkt kann zusätzlich durch Bestrahlung sterilisiert sein, um absolute Keimfreiheit bei der Benutzung zu gewährleisten. Die Herstellung des erfindungsgemäßen Zahnaufsatzkissens erfolgt vorzugsweise durch Spritzgießen. Ein Beispiel für geeignetes Material wird unter der Bezeichnung Elastosil LR5040/20 von der Firma Wacker Chemie angeboten. Einzelheiten über das beispielhafte Elastosil LR 5040/20 A/B sind dem Datenblatt der Firma Wacker Chemie zu entnehmen. Ein geeignetes Silikon kann aber auch von anderen Herstellern bezogen werden.

Das Zahnaufsatzkissen wird zudem mit jeweils gefasten oder gerundeten Kanten gefertigt.

Das erfindungsgemäße Zahnaufsatzkissen eignet sich besonders zur Einstiegsbehandlung einer craniomandibulären Therapie, bei Reizzuständen des Kiefergelenks sowie der umgebenden Muskulatur, einschließlich der Schulter-, Nacken- und Rückenmuskulatur, sowie zur Entspannung und somit zur Schmerzreduktion. Durch Platzierung der Zahnaufsatzkissen beidseitig auf den Zähnen des Unterkiefers, je nach Wohlbefinden im Vorderen oder auch hinteren Bereich des Kiefers, wird eine sanfte Dehnung des Kiefergelenks und der Kiefermuskulatur erzielt, was beim Benutzer oder Patienten zu einem Entspannungszustand führt. Als weitere neurophysiologische Folge wird der Vagus Nerv aktiviert, was zu einer Entspannung der Muskulatur im Schulter-, Nacken- und Rückenbereich führt. Durch die sanfte Dehnung des Kiefergelenks besteht zudem die Möglichkeit einer Beruhigung eines ggf. gereizten Kapselbandapparats, wobei sich auch der Diskus des Gelenks ggf. bei Dislokation mit begleitenden Maßnahmen an seinen vorgesehenen Platz zurückbewegen lässt, was zu einer Schmerzbeseitigung beiträgt.

Die erfindungsgemäßen Zahnaufsatzkissen können dabei zu einer begleitenden Vorbehandlung einer nachfolgenden Behandlung zur therapeutischen Dehnung des Kiefergelenks mittels kurzer Aufsatzkissen für die Backenzähne benutzt werden.

## Patentansprüche

1. Zahnaufsatzkissen (10) mit einer zum Aufsatz auf einen oder mehrere nebeneinanderliegende Zähne eines Benutzers geeignet ausgebildeten Form und Größe,
mit einem zur Auflage auf einer Zahnoberfläche des einen oder der mehreren Zähne ausgebildeten Grundkörper (12) und zwei seitlichen, vom Grundkörper (12) ausgehenden, und sich über die Länge des Grundkörpers erstreckenden Seitenwangen (14, 16),
**dadurch gekennzeichnet, dass** die Seitenwangen (14, 16) sich jeweils zu beiden Seiten des Grundkörpers erstrecken, und die derart angeordnet sind, dass sich ein zur Aufnahme eines oder mehrerer nebeneinanderliegender Zähne geeigneter Graben zu beiden Seiten des Grundkörpers zwischen den Seitenwangen (14, 16) und dem Grundkörper ergibt, wobei der Grundkörper eine Länge (L) entlang der Richtung des Grabens von 20 mm bis 35 mm aufweist.

2. Zahnaufsatzkissen gemäß Anspruch 1, wobei der Grundkörper eine Dicke von 2 bis 8 mm aufweist, die in einer Richtung senkrecht zu einer Fläche, mit der der Grundkörper auf einen Zahn aufsetzbar ist, gemessen wird.

3. Zahnaufsatzkissen gemäß Anspruch 1 oder 2, wobei die Seitenwangen (14, 16) eine Dicke von 1 bis 5 mm aufweisen, die in einer Richtung parallel zu einer Fläche, mit der der Grundkörper auf einen Zahn aufsetzbar ist, gemessen wird.

4. Zahnaufsatzkissen nach einem der vorhergehenden Ansprüche, das einstückig ausgebildet ist und aus medizinischem Silikonmaterial besteht.

5. Zahnaufsatzkissen nach Anspruch 4, wobei das Silikonmaterial eine Shore-A-Härte von 10 bis 30, bevorzugt von 15 bis 25, und besonders bevorzugt von 18 bis 22 aufweist.

6. Zahnaufsatzkissen nach einem der Ansprüche 1 bis 3, das einstückig aus einem Material, vorzugsweise medizinisches Silikon, ausgebildet ist, das eine Shore-A-Härte von 30 bis 70, bevorzugt von 45 bis 55, und besonders bevorzugt von 50 aufweist.

7. Zahnaufsatzkissen nach einem der vorhergehenden Ansprüche, wobei die Länge (S), mit der die Seitenwangen vom Grundkörper abstehen, zwischen 3 und 10 mm, bevorzugt zwischen 4 und 6 mm, beträgt.

8. Zahnaufsatzkissen nach einem der vorhergehenden Ansprüche, wobei das Verhältnis der Länge (L) des Grundkörpers zur Breite (B) des Zahnaufsatzkissens zwischen 1.4 und 1.8, insbesondere bei ca. 1.6, liegt.

9. Zahnaufsatzkissen nach einem der vorhergehenden Ansprüche, wobei das Verhältnis der Länge (L) der Seitenwangen zur Höhe (H) der Seitenwangen zwischen 1.4 und 1.8, insbesondere bei ca. 1.6, liegt.

## Claims

1. Dental bite cushion (10) with a shape and size formed to be suitable for placement onto one or more adjacent teeth of a user,
comprising a base body (12) formed for placement onto a tooth surface of the one or more teeth, and two lateral side wings (14, 16) protruding from said base body (12) and extending over the length of said base body,
**characterized in that** the side wings each extend on either side of said base body, and are arranged such that a trench suitable for receiving one or more adjacent teeth is formed on either side of said base body between said side wings (14, 16) and said base body, wherein said base body has a length (L) along the direction of said trench of 20 mm to 35 mm.

2. Dental bite cushion according to claim 1, wherein said base body has a thickness of 2 to 8 mm, measured in a direction perpendicular to a surface with which said base body can be placed onto a tooth.

3. Dental bite cushion according to claim 1 or 2, wherein said side wings (14, 16) have a thickness of 1 to 5 mm, measured in a direction parallel to a surface with which said base body can be placed onto a tooth.

4. Dental bite cushion according to one of the preceding claims, which is formed integrally and consists of medical-grade silicone material.

5. Dental bite cushion according to claim 4, wherein said silicone material has a Shore A hardness of 10 to 30, preferably of 15 to 25, and particularly preferably of 18 to 22.

6. Dental bite cushion according to one of the claims 1 to 3, which is formed integrally from a material, preferably medical-grade silicone, having a Shore A hardness of 30 to 70, preferably of 45 to 55, and more preferably of 50.

7. Dental bite cushion according to one of the preceding claims, wherein the length (S) by which said side wings protrude from said base body is between 3 and 10 mm, preferably between 4 and 6 mm.

8. Dental bite cushion according to one of the preceding claims, wherein the ratio of the length (L) of said base body to the width (B) of said dental bite cushion is between 1.4 and 1.8, is in particular approximately 1.6.

9. Dental bite cushion according to one of the preceding claims, wherein the ratio of the length (L) of said side wings to the height (H) of said side wings is between 1.4 and 1.8, is in particular approximately 1.6.

## Revendications

1. Coussinet de fixation dentaire (10) présentant une forme et une taille qui sont conçues pour être appropriées pour une fixation à une ou plusieurs dents adjacentes d'un utilisateur, avec un corps de base (12) qui est conçue pour reposer sur une surface de dent des une ou plusieurs dents et deux joues latérales (14, 16) qui sortent du corps principal (12) et s'étendent sur la longueur du corps principal, **caractérisé en ce que** les deux joues latérales (14, 16) s'étendent respectivement sur les deux côtés du corps principal et sont agencées de telle sorte qu'une tranchée soit produite, qui est adaptée pour recevoir une ou plusieurs dents adjacentes des deux côtés du corps principal entre les joues latérales (14, 16) et le corps principal, dans lequel le corps principal présente une longueur (L) de 20 mm à 35 mm le long de la direction de la tranchée.

2. Coussinet de fixation dentaire selon la revendication 1, dans lequel le corps principal présente une épaisseur de 2 à 8 mm, qui est mesurée dans une direction perpendiculaire à une surface avec laquelle le corps principal peut reposer sur une dent.

3. Coussinet de fixation dentaire selon la revendication 1 ou 2, dans lequel les joues latérales (14, 16) présentent une épaisseur de 1 à 5 mm qui est mesurée dans une direction parallèle à une surface avec laquelle la partie principale peut reposer sur une dent.

4. Coussinet de fixation dentaire selon l'une quelconque des revendications précédentes, qui est formé d'une seule pièce et est composé d'un matériau de silicone de qualité médicale.

5. Coussinet de fixation dentaire selon la revendication 4, dans lequel le matériau de silicone présente une dureté Shore A de 10 à 30, de préférence de 15 à 25, et de manière particulièrement préférée de 18 à 22.

6. Coussinet de fixation dentaire selon l'une quelconque des revendications 1 à 3, qui est formé d'une seule pièce en un matériau, de préférence du silicone médical, qui présente une dureté Shore A de 30 à 70, de préférence de 45 à 55, et de manière particulièrement préférée de 50.

7. Coussinet de fixation dentaire selon l'une quelconque des revendications précédentes, dans lequel la longueur (S) dont les joues latérales dépassent du corps principal est comprise entre 3 et 10 mm, de préférence entre 4 et 6 mm.

8. Coussinet de fixation dentaire selon l'une quelconque des revendications précédentes, dans lequel le rapport de la longueur (L) du corps principal à la largeur (B) du coussinet de fixation dentaire est compris entre 1,4 et 1,8, en particulier est d'environ 1,6.

9. Coussinet de fixation dentaire selon l'une quelconque des revendications précédentes, dans lequel le rapport de la longueur (L) des joues latérales à la hauteur (H) des joues latérales est compris entre 1,4 et 1,8, en particulier est d'environ 1,6.
